# EUROPÄISCHE PATENTSCHRIFT

(11) **EP 2 632 524 B1**
(45) Veröffentlichungstag und Bekanntmachung des Hinweises auf die Patenterteilung: **12.10.2016**
(21) Anmeldenummer: 11808151.2
(22) Anmeldetag: 14.10.2011
(51) Int. Cl.: A61M 16/04, A61F 5/443, A61F 13/02

(54) **HALSPFLASTER FÜR TRACHEALKANÜLEN ODER KÜNSTLICHE NASEN MIT SPRECHVENTIL**
NECK PATCH FOR TRACHEAL CANNULAS OR ARTIFICIAL NOSES HAVING A SPEAKING VALVE
PANSEMENT POUR LE COU DESTINÉ À DES CANULES TRACHÉALES OU DES NEZ ARTIFICIELS COMPORTANT UNE MEMBRANE VOCALE

(30) Priorität: 29.10.2010 DE 102010049895
(43) Veröffentlichungstag der Anmeldung: 04.09.2013
(73) Patentinhaber: Primed Halberstadt Medizintechnik GmbH, 28820 Halberstadt (DE)
(72) Erfinder: LEIBITZKI, Harry, 38889 Blankenburg (DE); SÜSS, Steffen, 38820 Halberstadt (DE)
(74) Vertreter: Donath, Dirk
(86) Internationale Anmeldenummer: PCT/DE2011/001867
(87) Internationale Veröffentlichungsnummer: WO 2012/055389

(56) Entgegenhaltungen:
- WO-A1-90/06735
- WO-A1-91/05579
- WO-A1-99/29268
- WO-A1-2004/000401
- WO-A1-2009/075636
- DE-U1-202007 010 008

## Beschreibung

Die vorliegende Erfindung betrifft ein Halspflaster für Trachealkanülen oder künstliche Nasen mit Sprechventil zum Aufkleben über ein Tracheostoma.

Tracheostoma- Prothesen (auch Trachealkanülen oder Tracheostoma-Tuben genannt) zur Behandlung kehlkopfloser (laryngektomierter) Patienten mit eröffneter Kehle (s.g. Tracheostoma) sind seit Jahrzehnten bekannt. Diese Prothesen gibt es als Ausführungen mit und ohne Sprechventil, wobei in den letzten Jahren die verschiedenen Ausführungsformen mit Sprechventil immer mehr an Bedeutung bei der Rehabilitation der laryngektomierten Patienten gewonnen haben, da durch sie Sprecherfolge bei den Patienten ermöglicht werden.

Die Tracheostoma- Prothesen werden in ein Tracheostoma eingesetzt und befestigt.

Als äußere Befestigung am Patienten dienen u.a. Haltebänder und Pflaster.

WO2009/075636 A1 offenbart eine Trachealkanülenbefestigung bestehend aus einem Flachteil mit einer zentralen Öffnung und einer Befestigungsfläche, wobei das Flachteil eine dünne, planare, flexible, einseitig klebende Folie mit einer Dicke zwischen 5 und 100µm, bevorzugt 10 bis 50µm und einen Stützrahmen aufweist.

DE 10 392 887 T5 offenbart eine Trachealkanülenbefestigung mit einem Flachteil, das mit einer Öffnung, durch welche eine Trachealkanüle hindurchgeführt und gleichzeitig gehaltert wird, sowie einer Befestigungsfläche versehen ist, welche ein klebriges Gelmaterial aufweist. Diese Befestigung dient dem Abdichten und Haltern der Kanüle gegenüber dem Tracheostoma.

Aus DE 20 2008 017 105 U1 ist eine Tracheostomastabilisierung zum Unterstützen der Befestigung von Kanülen, Filtern und Ventilen über einem Stroma als allgemein planares Flachteil, das mit einer Öffnung versehen ist, die nicht zur Tracheostoma- Kanülenbefestigung dient, bekannt, bei der das in der Nachbarschaft des Tracheostomas mit einer Klebfolie auf die Haut zu klebende Flachteil zum seitlichen Abdichten der Unebenheiten weich und elastisch ausgebildet ist.

Diese weiche und elastische Funktion unter der Klebfolie wird durch einen kreisrunden Schaumstoff, welcher mit einer harten, runden Kunststoffscheibe stoffschlüssig verbunden ist, generiert.

Dabei ist die Klebfolie über der harten Kunststoffscheibe als Pflaster mit Aufnahmering für Kanülen, Ventile, Filter usw. ausgebildet. Das Pflaster ist mit der Haut des Patienten verbindbar.

Gemäß DE 20 2008 017 105 U1 ist das Flachteil 3-5 mm dick und die Klebfolie doppelseitig klebend.

Der Nachteil dieser technischen Lösung ist, dass die Kunststoffscheibe recht starr und nicht atmungsaktiv und durch den Schaumstoff eine erhöhte Bauform besteht, so dass der Tragekomfort für den Patienten nicht besonders hoch ist.

Der Erfindung liegt die Aufgabe zugrunde, ein Halspflaster zum Aufkleben über ein Tracheostoma anzugeben, welches sehr leicht ist, eine gute Atmungsaktivität und ein sehr gutes Abdichtverhalten bei bestimmungsgemäßen Aufkleben über einem Tracheostoma im Hals eines Patienten aufweist.

Darüber hinaus soll eine geringe Bauhöhe erreicht werden, was neben der guten Atmungsaktivität den Tragekomfort für den Patienten wesentlich verbessert.

Gelöst wird diese Aufgabe durch ein Halspflaster gemäß dem ersten Patentanspruch. Vorteilhafte Ausgestaltungen der Erfindung sind in den nachgeordneten Ansprüchen angegeben.

Das Wesen der Erfindung besteht in der Bereitstellung eines Halspflasters in Form einer dünnen, planaren, flexible Folie mit einer Dicke zwischen 5 und 30µm, welche auf ihre proximalen Seite (der dem Patienten zugewandten Seite) einseitig klebend ist und auf ihrer distalen (dem Patienten abgewandten Seite) ein zentrisch angeordnetes Loch, um welches eine harte, runde Kunststoffscheibe (Konnektor) mit zentrischer Ausnehmung angeordnet ist, sowie einen Stützrahmen aufweist, wobei der Stützrahmen den äußeren Rand der Folie umgibt und etwa 5 bis 10-fach dicker als die Folie ist, so dass eine Positionierung des Halspflasters ohne Zusammenrollen möglich ist.

Die Folie ist vorteilhaft als Elastomerfolie, bspw. TPU (thermoplastes Uretan) oder TPE (thermoplastes Polyethylen) ausgeführt.

Die Folie und der Rahmen können ein- oder zweistückig ausgestaltet sein und eine rechteckige oder ovale Form aufweisen.

Die Kunststoffscheibe / der Konnektor besteht aus einem gegenüber der Folie und dem Rahmen wesentlich härterem Kunststoff-Material, wie bspw. Silicon, PVU, PET, PU oder PE), oder metallischem Material und weist die Funktion eines Schraub-, Bajonett- oder Klemm- Verschlusses auf. Vermittels dieses Verschlusses sind diverse Bauteile, wie bspw. Trachealkanülen, künstliche Nasen, Ventile, etc. halterbar.

Der Vorteil der erfindungsgemäßen Halspflasters ist die gute Atmungsaktivität und das sehr gute Abdichtverhalten bei bestimmungsgemäßen Einsatz des Aufklebens der sehr dünnen Folie über einem Tracheostoma im Hals eines Patienten bei gleichzeitiger guter Halterung der Bauteile an der Kunststoffscheibe / dem Konnektor des Pflasters.

Besonders von Vorteil ist die Tatsache, dass bei dem erfindungsgemäßen Halspflaster eine harte Kunststoffscheibe mit Schaumstoffpolsterung, wie sie aus dem Stand der Technik bekannt ist, nicht zum Einsatz kommt, wodurch die Bauhöhe (erfindungsgemäßes Halspflaster + weiteres Bauteil, wie bspw. einer sehr flachen laterale Nase mit 5 mm Höhe) sehr gering gehalten werden kann, was neben der guten Atmungsaktivität den Tragekomfort für den Patienten wesentlich verbessert.

Die Erfindung wird nachstehend an Hand des Ausführungsbeispiels und der Figur näher erläutert. Dabei zeigt:

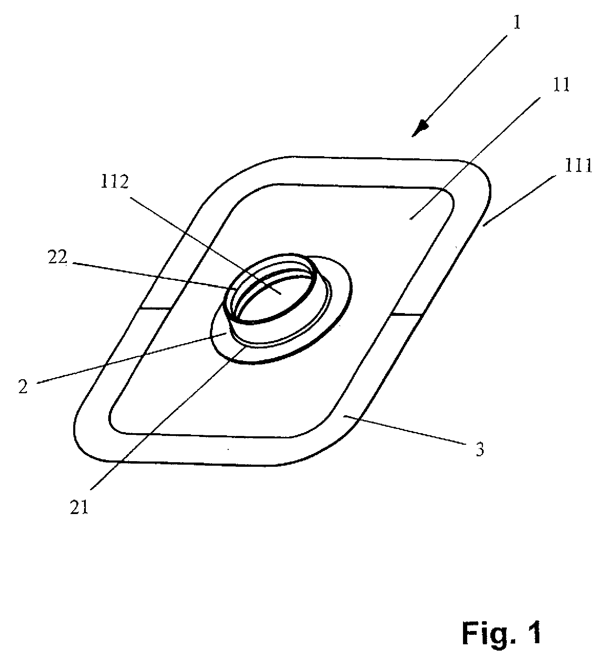

- Fig. 1:: eine schematische 3-D-Darstellung einer Ausführungsform des erfindungsgemäßen Halspflasters.

Fig. 1 zeigt ein Halspflaster (1) in Form einer dünnen, planaren, flexible Folie (11) mit einer Dicke zwischen 5 und 30µm, welche auf ihre proximalen Seite (der dem Patienten zugewandten Seite- 111) einseitig klebend ist und auf ihrer distalen (dem Patienten abgewandten Seite) ein zentrisch angeordnetes Loch (112), um welches eine harte, runde Kunststoffscheibe (2) / (Konnektor- 2) mit zentrischer Ausnehmung (21) angeordnet ist, sowie einen Stützrahmen (3) aufweist, wobei der Stützrahmen (3) den äußeren Rand der Folie umgibt und etwa 5 bis 10-fach dicker als die Folie (11) ist, so dass eine Positionierung des Halspflasters (1) ohne Zusammenrollen möglich ist.

Die Folie (11) ist als Elastomerfolie aus thermoplastem Uretan ausgeführt.

Die Folie (11) und der Stützrahmen (3) können ein- oder zweistückig ausgestaltet sein.

Die Kunststoffscheibe (2) / (Konnektor- 2) besteht aus Silicon mit einem Halteverschlussrand (22). Vermittels dieser Kunststoffscheibe / dieses Konnektors (2) ist eine flache laterale künstliche Nasen halterbar.

### Bezugszeichenliste

- 1: - Halspflaster
- 11: - Folie
- 111: - proximalen Seite
- 112: - Loch
- 2: - Kunststoffscheibe / Konnektor
- 21: - Halterrad
- 22: - Halteverschlussrand
- 3: - Stützrahmen

## Patentansprüche

1. Halspflaster zum Aufkleben auf ein Tracheostoma bestehend aus einem Flachteil, das mit einer Öffnung, durch welche eine Trachealkanüle hindurchführbar ist, sowie einer Befestigungsfläche versehen ist, welche ein klebrige Funktionalität aufweist, wobei das Flachteil eine dünne, planare, flexible Folie (11) mit einer Dicke zwischen 5 und 30µm ist, welche auf ihrer proximalen Seite (111) einseitig klebend ist und auf ihrer distalen Seite ein zentrisch angeordnetes Loch (112), um welches eine runde Kunststoffscheibe / ein Konnektor (2) mit zentrischer Ausnehmung (21) angeordnet ist, und das Flachteil einen Stützrahmen (3) aufweist, wobei der Stützrahmen (3) den äußeren Rand der Folie umgibt, so dass eine Positionierung des Halspflasters (1) ohne Zusammenrollen möglich ist, **dadurch gekennzeichnet, dass** der Stützrahmen (3) etwa 5 bis 10- fach dicker als die Folie (11) ist, die Folie (11) eine Elastomerfolie aus thermoplastem Kunststoff ist und die Kunststoffscheibe (2) / der Konnektor (2) aus einem gegenüber der Folie (11) und dem Rahmen wesentlich härterem Kunststoff- Material mit einem Halteverschlussrand (22) besteht.

2. Halspflaster zum Aufkleben auf ein Tracheostoma gemäß Anspruch 1, **dadurch gekennzeichnet, dass** die Folie (11) und der Stützrahmen (3) ein- oder zweistückig ausgestaltet sind.

3. Verwendung eines Halspflaster zum Aufkleben auf ein Tracheostoma gemäß einem der Ansprüche 1 und 2 zum Haltern von Trachealkanülen oder künstlichen Nasen mit und ohne Sprechventil.

## Claims

1. A neck patch to stick on a Tracheostoma consisting of a flat part which is provided with an opening, through which a tracheal cannula can be passed, and an attachment surface, which has an adhesive functionality, the flat part is a thin, planar, flexible film (11) with a thickness of between 5 and 30 µm, which is adhesive on one side on its proximal side (111) and on its distal side it has a centrically arranged hole (112) surrounded by a round plastic disk (2) with a centric recess (21), and the flat part comprises a supporting frame (3) that surrounds the outer edge of the film (11) so that it is possible to position the neck patch (1) without rolling it up, **characterized in that** the supporting frame (3) is approximately five to ten times thicker than the film (11), the film is an elastomer film made of thermoplastic plastics, and the plastic disk (2)/the connector (2) is made of a considerably harder material than the film (11) and the frame (3) and comprises a retaining closure edge (22).

2. Neck patch to stick on a tracheostoma according to claim 1, wherein the film (11) and the supporting frame (3) are made either in one piece or in two pieces.

3. Use of a neck patch to stick on a tracheostoma according to one or several of the previous claims for holding tracheal cannulas or artifcial noses with and without a speaking valve.

## Revendications

1. Pansement pour le cou à coller sur une trachéostomie qui se compose d'une partie plate munie d'une ouverture pour faire passer une canule trachéale ainsi que d'une surface de fixation ayant une fonctionnalité adhérente, la partie plate étant une mince feuille plane et flexible (11) avec une épaisseur entre 5 et 30µm, adhésive unilatéralement sur son côté proximal (111) et présentant sur son côté distal, un trou disposé central (112) autour duquel un disque de plastique (2) rond comportant un évidement central (21) est disposé, et la partie plate étant munie d'un cadre support (3), le cadre support (3) entourant le bord extérieur de la feuille de telle manière qu'un positionnement du pansement pour le cou (1) peut être réalisé sans enroulement, est **caractérisé en ce que** le cadre support est 5 à 10 fois plus épais que la feuille (11), la feuille (11) étant une feuille élastomère en thermoplaste et disque de plastique (2) / le connecteur (2) se composant d'une matière sensiblement plus dure que la feuille (11) et le cadre et étant muni d'un bord d'étanchéité (22).

2. Pansement pour le cou à coller sur une trachéostomie suivant la revendication 1 est **caractérisé en ce que** la feuille (11) et le cadre support (3) sont constitués d'une ou de deux partie.

3. Utilisation d'un pansement pour le cou à coller sur une trachéostomie suivant une ou plusieurs des revendications précédentes pour maintenir des canules trachéales ou des nez artificiels avec ou sans une membrane vocale.
